# EUROPEAN PATENT SPECIFICATION

(11) **EP 2 163 274 B1**
(45) Date of publication and mention of the grant of the patent: **23.05.2012**
(21) Application number: 08764872.1
(22) Date of filing: 23.05.2008
(51) Int. Cl.: A61M 16/04, A61B 1/00, A61B 1/267, A61B 1/273, A61B 17/24

(54) **ENDOTRACHEAL TUBE INSERTION ASSIST INSTRUMENT**
INSTRUMENT ZUR ERLEICHTERUNG DER EINFÜHRUNG EINES ENDOTRACHEALTUBUS
INSTRUMENT AIDANT A INSERER UN TUBE TRACHEAL

(30) Priority: 25.05.2007 JP 2007138473
(43) Date of publication of application: 17.03.2010
(73) Proprietor: Senko Medical Instrument Manufacturing Co., Ltd., Bunkyo-ku Tokyo 113-0033 (JP)
(72) Inventor: UESUGI, Takanobu, Osaka 5620003 (JP)
(74) Representative: Horn Kleimann Waitzhofer
(86) International application number: PCT/JP2008/059953
(87) International publication number: WO 2008/146893

(56) References cited:
- GB-A- 2 096 467
- JP-A- 01 158 931
- JP-A- 01 503 365
- JP-A- 2002 505 925
- US-A1- 2006 004 258

## Description

### Technical Field

The present invention reflates to an endotracheal intubation assist instrument for inserting an endotracheal tube into a patient's trachea, when artificial respiration is required for the patient who has an airway difficultly The airway difficulty is the leading cause of death during a general anesthesia operation. For example, the present invention can be applied to a tool for assisting the endotracheal intubation under the bronchofiberscope observation which is used for the patient having a severe difficulty in the endotracheal intubation.

### Background Art

An endotracheal intubation is required in a medical rescue scene for managing the airway maintenance of the patient who has an airway difficulty. For example, the endotracheal intubation is required for the patient whose airway is collapsed by an accident, the patient who cannot manage breathe by himself in a coma state or in a drunk state, the patient whose spontaneous breathing is decreased or stopped in the general anesthesia operation, the patient who needs a stent placement operation to the bronchus and so on. In the prior art, an oral endotracheal intubation for inserting the endotracheal tube from the oral cavity to the trachea and a nasotracheal intubation for inserting the endotracheal tube from the nasal cavity to the trachea are known for the endotracheal intubation.

In the conventional endotracheal intubation, the laryngoscope or the light-guided type stylet generally is used. For example, the endotracheal tube is inserted into the laryngeal cavity by observing the glottis with the laryngoscope and then the endotracheal tube is inserted further into the trachea through the glottis.

It is difficult to find the glottis which is the entrance of the patient's trachea in the conventional endotracheal intubation, either by the oral or by the nasotracheal intubation method. In the human body, the esophagus and the trachea are separated near at the larynx and the pharynx. When the endotracheal tube is inserted from the oral cavity and the endotracheal tube is kept on going down along the throat, naturally it will be lead to the esophagus in the most cases. The trachea is located in the chest side with a certain angle against the esophagus and the trachea curves by a certain angle. The curve angle of the trachea varies among individuals, in addition, the figure of the human organs from the oral cavity to the trachea where the endotracheal tube will pass have various shapes. The shape of the oral cavity, the shape of the tongue, the shape of the pharynx, the shape of the epiglottis, and the shape of the laryngeal cavity vary among individuals. It is not easy for the operator to insert the endotracheal tube to the trachea appropriately in a short time when airway management is required during the emergency situation.

In the prior art, the operation of the endotracheal intubation under the bronchofiberscope observation for inserting the endotracheal tube from the oral cavity to the trachea by using the bronchofiberscope is the safest and surest endotracheal intubation method for the patient having severe difficulty with the endotracheal intubation.

The bronchofiberscope is a fiberscope whose diameter is about 5 mm. It is inserted from the oral cavity or the nasal cavity and it is kept on going down along the throat to find out the glottis by observing the surrounding organs directly. It is inserted further into the trachea to find out and verify the trachea position. In the conventional endotracheal intubation under the bronchofiberscope observation, after the insertion of the bronchofiberscope, the endotracheal tube is inserted to the trachea utilizing the bronchofiberscope tube as a stylet of the endotracheal tube.

In the prior art, the guidewire method is known. The wire is stuck and injected into the trachea from the cricothyroid membrane directly, the wire is pulled from the inside of the trachea up to the oral cavity, then the endotracheal tube is inserted from the oral cavity to the trachea utilizing the wire as a guide line of the endotracheal tube (JPA2003-235978).
The prior art 1 : Japanese laid open patent application JPA2002-505925
The prior art 2 : Japanese laid open patent application JPA2003-235978

### Disclosure of Invention

### The problems to be solved

In the conventional endotracheal intubation method under the bronchofiberscope observation, the bronchofiberscope is used as a guide line for inserting the endotracheal tube into the trachea, so it works as an endotracheal intubation assist instrument. It is difficult to insert the endotracheal tube to the trachea by seeking the trachea directly, so the two step procedure is known, the first step is that the bronchofiberscope is inserted from the oral cavity to the trachea, the second step is that the endotracheal tube is inserted to the trachea by utilizing the bronchofiberscope as a guide line for the endotracheal tube.

However, it is difficult for the operator to control and insert the bronchofiberscope from the oral cavity to the trachea even though he can observe organs in the bronchofiberscope view field.

The first problem is that the bronchofiberscope view field often is blocked by the obstacles in the bronchofiberscope insertion operation. There are a lot of obstacles present in the route from the oral cavity to the trachea. For example, the tongue, the saliva, and the epiglottis can be the obstacle that blocks the bronchofiberscope view field. The view field obtained by the bronchofiberscope is narrow and limited on the front edge of the fiber. Therefore, the view field is blocked easily by the organs present on the way to the trachea.

The second problem is that the skilled hand is required for the bronchofiberscope operation. The bronchofiberscope can have its position and angle controlled by the operator, but the actual controllable portion is limited to about 2 cm at the front edge portion of the fiberscope and the controllable direction is limited to bend and back around the front space. Furthermore, the controllable angle is limited up to 90 degrees. The total length of the bronchofiberscope is several dozen centimeters. It is a comparably long tool, but the controllable portion is limited to the front edge portion of the fiberscope, so the usability is not enough for the operator.

Next, the conventional bronchofiberscope has a problem in its structure strength. The shape of the bronchofiberscope is not stable enough because the bronchofiberscope is made of flexible material. Because of the bronchofiberscope is made of flexible and elastic material, it cannot go forward by pushing the obstacles away, so it cannot go in the desirable direction. Therefore, the bronchofiberscope should take a roundabout route even if the obstacles are small, so the operation of the bronchofiberscope will be difficult.

Next, there is a problem that the structure of the airway the bronchofiberscope goes through varies due to the differences among individuals. There are certain structural differences in the oral cavity, the pharynx, the larynx and the trachea among individuals. In addition these organs can be deformed due to the surgery in the past, and furthermore these organs can be deformed due to the severe damages by the accident. Especially, in the case that the patient cannot move the head and neck due to the loss of the neck extensor by the cervical spine injury by the accident, the bronchofiberscope operation condition becomes severe because the treatment for adjusting the position of the patient's head and neck to make an easier bronchofiberscope operation cannot be accommodated in this situation. As mentioned above, the status of the airway and the status of the patient are different among individuals, so it is difficult for the operator to operate the bronchofiberscope with narrow view field in various states of the airway.

In conclusion, the bronchofiberscope operation for inserting from the oral cavity to the trachea via the bronchia is very difficult, so the highly skilled hand is required for every operator.

In the prior art, as shown in the prior art 1 of the Japanese laid open patent application JPA2002-505925, a variety of devices are added to the endotracheal tube. However, that prior art cannot solve those problems of the conventional endotracheal intubation but only disclose the method that can be applied to the after treatment for the lungs where the endotracheal intubation is conducted. For example, the after treatment for the lungs is the treatment for securing the airway to the one lung while the other lung takes another treatment. According to the Japanese laid open patent application JPA2002-505925 shown in Fig. 22 of this specification, the device added to the bronchofiberscope is applied to the treatment for the lungs after the endotracheal intubation.

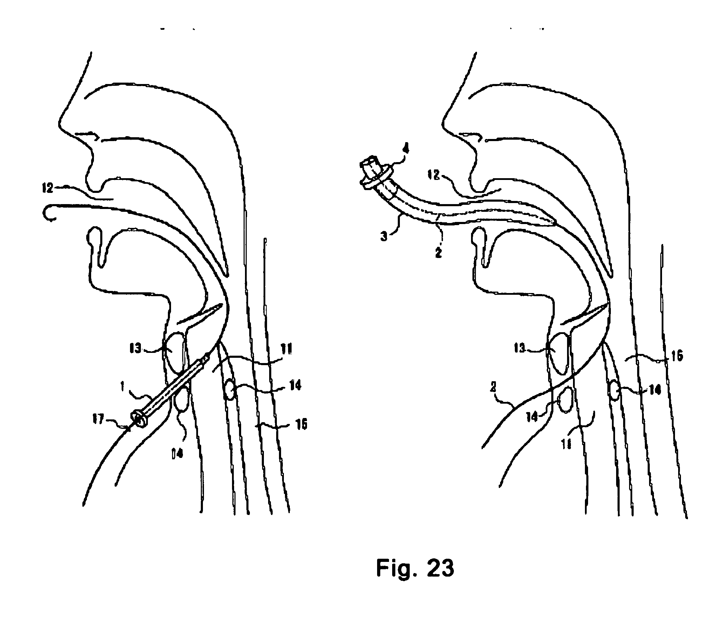

Next, the prior art 2 of the Japanese laid open patent application JPA2003-235978 is provided as the solution for the endotracheal intubation, and a thin wire is stuck and injected into the trachea from the body surface by the injection tool, so the operation for pulling the wire from the inside of the trachea through the glottis, the larynx, the pharynx up to the oral cavity as shown in Fig. 23 is not easy.

Instruments representing the preamble of claim 1 are disclosed in documents GB-2 096 467 and US 2006/004 258.

It is an object of the present invention to provide an endotracheal intubation assist instrument for inserting the bronchofiberscope from the oral cavity to the trachea easily and then inserting the endotracheal tube into the trachea easily even if the operator does not have a highly skilled hand for bronchofiberscope operation.

### Means for solving the problem

In order to achieve the above-mentioned object, an endotracheal intubation assist instrument according to the present invention comprises: a guide tube having flexibility and a gently curved outline; a side hole arranged in a wall of the guide tube near the front edge on the inner circumference side of the curved outline; wherein the front edge of the guide tube is insertable from the oral cavity or the nasal cavity up to a vicinity of the entrance of the esophagus, a bronchofiberscope is insertable from the terminal end opening of the guide tube up to the side hole, and the view field of the bronchofiberscope can be ensured via the side hole so that the trachea can be ascertained easily.

According to the above-mentioned configuration of the endotracheal intubation assist instrument, first the front edge of the guide tube goes naturally from the oral cavity to the esophagus, which can be found easily. Then the guide tube inserted into the entrance of the esophagus is pulled back slowly, so the glottis and the trachea can be found easily by the view field of the bronchofiberscope via the side hole. When the operator finds the glottis and trachea, the operator operates the bronchofiberscope to go forward to the trachea, and the front edge of the bronchofiberscope can reach the trachea easily.

The endotracheal intubation assist instrument further comprises a cut assisting structure for assisting in cutting the guide tube from the terminal end opening up to the side hole. After inserting the bronchofiberscope to the trachea, the guide tube is removed more easily by cutting the guide tube from the terminal end opening up to the side hole with the cut assisting structure and the bronchofiberscope remains in the trachea as it is.

According to the above-mentioned configuration, the guide tube should be removed after the bronchofiberscope is inserted to the trachea, if not, the guide tube will be the obstacle for the endotracheal tube so the endotracheal tube cannot insert to the trachea. The cut assisting structure can assist operator to cut only the guide tube from the terminal end opening up to the side hole and remove it away as the bronchofiberscope remains in the trachea.

For example, it is preferable that the cut assisting structure is a cutting line provided from the terminal end opening of the guide tube up to the side hole. According to the above configuration, the operator can cut the guide tube along the cutting line easily.

It is also preferable that the cut assisting structure comprises a cut gap provided in advance from the terminal end opening of the guide tube up to the side hole, and a cover film to cover the cut gap. According to the above configuration, the operator can cut the guide tube along the cut gap by breaking the cover film easily.

Next, it is preferable that the front edge of the side hole has a ramp part as a base for the bronchofiberscope. While the guide tube goes forward from the oral cavity to the esophagus and goes backward from the esophagus to the oral cavity, the operator can find the glottis by the bronchofiberscope easily by utilizing the base. In addition, the operator can operate the bronchofiberscope to go forward to the entrance of the trachea easily by utilizing the base. The front edge of the side hole has a ramp part as a base, the front edge of the bronchofiberscope is set on the base, and the view field from the front edge of the bronchofiberscope can be ensured obliquely in front of the side hole.

According to the above configuration, the view field from the front edge of the bronchofiberscope becomes oblique in front via the side hole. The trachea can be found easily because the trachea is located in the chest side with a certain angle against the esophagus. In addition, the base can adjust the angle of the front edge of the bronchofiberscope to go to the trachea easily.

For example, it is preferable that the oblique angle of the ramp part of the base is from 30 degrees to 60 degrees relative to the guide tube axis direction.

According to the configuration, the angle of the front edge of the bronchofiberscope becomes appropriately to go to the trachea.

Next, it is preferable that the endotracheal intubation assist instrument further comprises a blue-green guide line indicating on the inner wall from the terminal end up to the front edge of the guide tube.

According to the configuration, if there is the blue-green line is indicated on the inner wall from the terminal end up to the front edge of the guide tube, the operator can recognize the direction of the side hole easily because the blue-green is the complementary color of blood red. If there is blood in the guide tube, the operator still can recognize the blue-green line easily.

Next, it is preferable that the endotracheal intubation assist instrument further comprises a grip at the terminal end of the guide tube.

According to the configuration, the operator can grip the guide tube more easily. In addition, the operator can operate the guide tube from the oral cavity up to the esophagus more easily, and pull and remove the guide tube by cutting it along to the cutting line easier.

It is preferable that the position of the side hole on the guide tube is about 30 mm to 80 mm from the front edge. It is expected that the position of the entrance of the trachea is about 30 mm to 80 mm backward from the front edge of the guide tube when the front edge of the guide tube is reached at the esophagus.

It is preferable that the length of the side hole is about 10 mm to 30 mm along the axis direction. It is expected that the operation of the length of the bronchofiberscope is not affected when the length of the side hole is about 10 mm to 30 mm considering the axis diameter and the motion range of the front edge of the bronchofiberscope. Regarding the width of the guide tube, the appropriate size will be fine in order to secure the appropriate space for bronchofiberscope operation.

Next, it is preferable that the guide tube further comprises a rotation torque transmitter that can transmit the rotation torque of the force applied to the terminal end of the guide tube up to the front edge.

According to the configuration, when it is necessary for the guide tube to rotate around the axis in order to rotate the side hole around the axis in order to find the glottis around the bronchia, the rotation torque is applied to the terminal end, the rotation torque is transmitted to the front edge by the rotation torque transmitter, so the rotate operation for the guide tube becomes easier. For example, the rotation torque transmitter is made of the wire or the plastic having an appropriate axial rigidity installed in the guide tube.

Next, it is preferable that the endotracheal intubation assist instrument further comprises an inflatable/deflatable balloon arranged near the front edge of the guide tube. The angle of elevation of the front edge of the guide tube against the bronchia wall can be adjusted by inflating the balloon.

According to the configuration, the angle of elevation of the front edge of the guide tube against the bronchia wall can be adjusted easily by inflating the balloon. When the trachea cannot be observed by the view field of the bronchofiberscope via the side hole, the angle of the front edge of the guide tube and the view field of the bronchofiberscope can be adjusted easily by controlling the inflation of the balloon, and the trachea will be found more easily.

### Brief Description of Drawings

Fig. 1 illustrates a basic structure of the first endotracheal intubation assist instrument 100.
Fig. 2 illustrates a vertical section view of procedure that the bronchofiberscope 200 is set into the inside of the endotracheal intubation assist instrument 100.
Fig. 3 illustrates the motion range of the front edge of the bronchofiberscope 200 set on the base 21.
Fig. 4 illustrates the vertical cross section view of the general structure of the oral cavity, the nasal cavity, the esophagus and the trachea.
Fig. 5 illustrates the view showing the appearance when the front edge of the endotracheal intubation assist instrument 100 reaches the entrance of the esophagus via the oral cavity.
Fig. 6 illustrates the view showing the appearance when the bronchofiberscope 200 is inserted into the inside of the endotracheal intubation assist instrument 100 in the state shown in Fig. 5.
Fig. 7 illustrates the view showing the appearance when the front edge of the bronchofiberscope 200 is put onto the base 21 of the side hole 20.
Fig. 8 illustrates the view showing the appearance when the guide tube 10 with the bronchofiberscope 200 in the states shown in Fig. 7 is pulled back slowly to find the glottis.
Fig. 9 illustrates the view showing the appearance when the operator operates the front edge of the bronchofiberscope 200 to go to the glottis.
Fig. 10 illustrates the view showing the appearance when the guide tube 10 is cut by the cutting line 13 and the only guide tube 10 is removed away.
Fig. 11 illustrates the view showing the appearance when the guide tube 10 is removed away completely.
Fig. 12 illustrates the view showing the appearance when the endotracheal tube 300 is inserted by utilizing the bronchofiberscope 200 as the guide line.
Fig. 13 illustrates the view showing the appearance when the endotracheal tube 300 is inserted completely
Fig. 14 illustrates the view showing the appearance when the bronchofiberscope 200 is removed away.
Fig. 15 illustrates the view showing the appearance when the bronchofiberscope 200 is removed away completely.
Fig. 16 illustrates a basic structure of the endotracheal intubation assist instrument 100a embodying the invention.
Fig. 17 illustrates the view showing the developed appearance of the inside wall of the guide tube 10b.
Fig. 18 illustrates a basic structure of the fourth endotracheal intubation assist instrument 100c.
Fig. 19 illustrates a basic structure of the fifth endotracheal intubation assist instrument 100d.
Fig. 20 illustrates a basic structure of the sixth endotracheal intubation assist instrument 100e.
Fig. 21 illustrates the view showing the appearance when the angle of elevation of the front edge of the guide tube 10e against the bronchia wall becomes large by inflating the balloon 30 through the air tube 19.

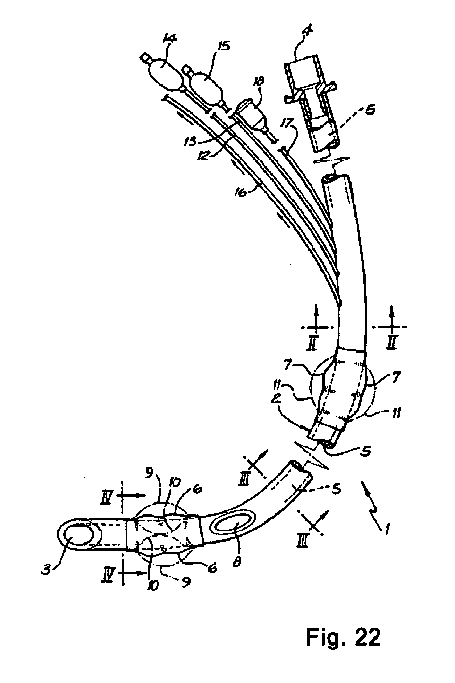

Fig. 22 illustrates the structure of the blocker tube shown in Japanese laid open patent application JPA2002-505925.
Fig. 23 illustrates the operation of the retrograde endotracheal intubation shown in Japanese laid open patent application JPA2003-235978.

### Best Mode for Carrying Out the Invention

Hereinafter, the example for carrying out the present invention will be described by way of embodiments. However, the present invention is not limited to the embodiments.

### (Embodiment 1)

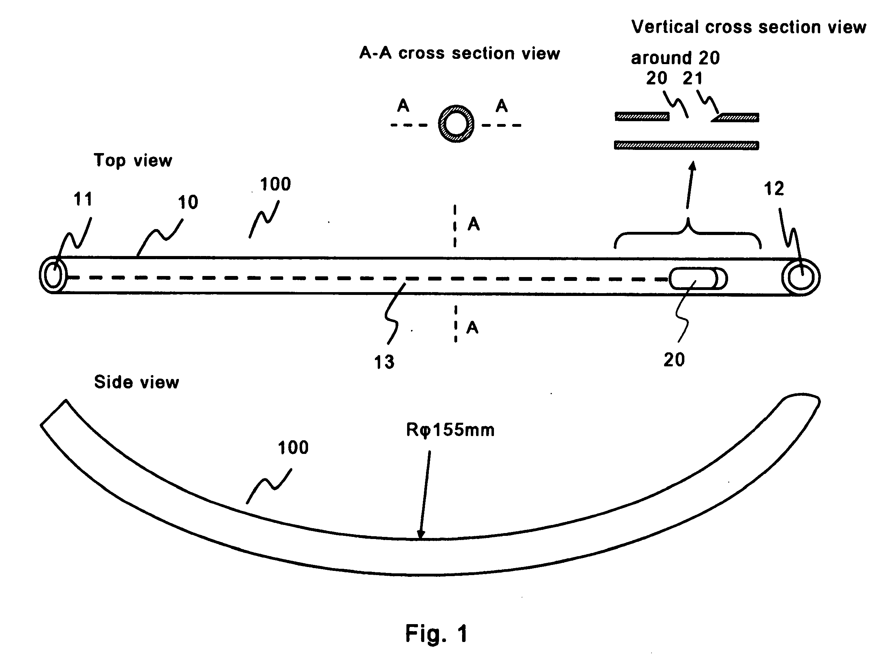

The first endotracheal intubation assist instrument 100 of this Embodiment 1 helpful for understanding the present invention is described below. Fig. 1 illustrates a basic structure of the first endotracheal intubation assist instrument 100. The central figure is the top view of the endotracheal intubation assist instrument 100, the lower figure is the right hand side view of the endotracheal intubation assist instrument 100. The upper figure is the cross section view along A-A line shown in the central figure and the vertical cross section view around the side hole 20. As shown in Fig. 1, the endotracheal intubation assist instrument 100 comprises a guide tube 10 and a side hole 20. The side hole 20 is provided as a part of the guide tube 10.

The guide tube 10 is made of material having both flexibility and appropriate structural strength. For example, it is made of a silicone tube. Flexibility is required for minimizing the damage to the patient's organs when the guide tube is inserted from the oral cavity or the nasal cavity, through the pharynx, the larynx, the glottis and up to the trachea. Appropriate structural strength is required for the guide tube. If the guide tube does not have appropriate structural strength, while the guide tube is inserted from the oral cavity or the nasal cavity through the pharynx, the larynx, the glottis up to the trachea, it cannot go through among those organs that will be obstacles for the guide tube insertion. Therefore appropriate structural strength is required.

The guide tube 10 has a gently curved outline. For example, it has 155mm radius of curvature in this configuration. The guide tube 10 is used for assisting insertion of the endotracheal tube into the oral cavity or the nasal cavity through the pharynx, the larynx, the glottis and up to the trachea, and therefore, the gently curved outline of the guide tube 10 is useful to follow through these organs. It is preferable that the shape of the front edge and the edge shape of the side hole 20 are round. The damage of the patient's organs will be decreased when the shape of the guide tube 10 and the edge shape of the side hole 20 are round because these parts contact with patient's organs directly. In addition, it is also preferable that the angle of the front edge has a certain skew but not right angle to the axis. When the shape of the front edge is round and the angle of the front edge has a certain skew, it is easier for the guide tube to be pushed among these organs.

There is an opening 11 at the terminal end of the guide tube 10, in addition there is a leading opening 12 at the front edge. As described later, the bronchofiberscope 200 is inserted into the guide tube 10 during the operation procedure using the endotracheal intubation assist instrument 100 of the present invention, if there is the leading opening 12 at the front edge of the guide tube 10, the operator can verify the position of the front edge of the guide tube 10 by observing via the leading opening 12 whether the front edge is in the esophagus or the trachea, the usability will be improved.

The guide tube 10 further comprises a cutting line 13 installed from the terminal end opening to the side hole 20 to cut easier when cutting and removing the guide tube. As described later, when the front edge of the bronchofiberscope 200 is inserted into the trachea, the purpose of the first endotracheal intubation assist instrument 100 of the present invention has already been achieved, and it is no longer needed for the following procedure of the endotracheal intubation stage, so the guide tube 10 should be removed. The guide tube 10 can be cut and separated along to the cutting line 13 during pulling back and it can be removed away.

Next, the side hole 20 is installed in the tube wall around the front edge of the guide tube 10.

It is preferable that the installed position of the side hole 20 on the guide tube 10 is about 30 mm to 80 mm from the front edge. The side hole 20 is the opening where the front edge of the bronchofiberscope 200 is located for securing the view field and verifying the position and the direction of the trachea, when the front edge of the guide tube 10 is located at about the entrance of the esophagus, then the side hole will be located at about the entrance of the trachea.

It is preferable that the length of the side hole 20 of the guide tube is about 10 mm to 30 mm along to the axis direction. The margin between the front edge and the side hole is necessary in order to avoid the influence on the operation of the front edge part of the bronchofiberscope 200. On the contrary, if the length of the side hole 20 is too large, the positioning control of the front edge part of the bronchofiberscope 200 becomes difficult. Therefore, the length of the side hole 20 should be within the above-mentioned range.

Regarding the width of the side hole 20, it is selected according to the diameter of the bronchofiberscope 200 with a certain margin. Most diameters of the bronchofiberscope 200 are less than 5 mm, therefore the reasonable range of the width of the side hole 20 is about 3 mm to 7 mm.

The side hole 20 has the base 21. The base 21 is a ramp part installed on the front edge wall of the side hole 20. When the front edge part of the bronchofiberscope 200 is set on the base 21, the view field from the front edge of the bronchofiberscope 200 becomes oblique due to the ramp part of the side hole 20.

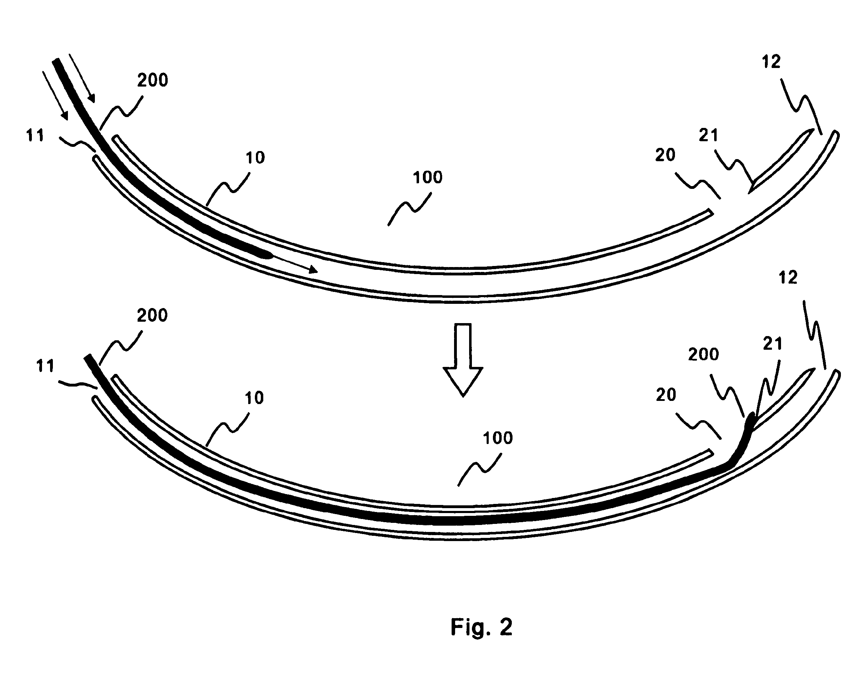

The first endotracheal intubation assist instrument 100 and the bronchofiberscope 200 are set as shown in Fig. 2. Fig. 2 illustrates a vertical section view of the procedure that the bronchofiberscope 200 is set into the inside of the endotracheal intubation assist instrument 100. During the operation of the bronchofiberscope 200 as described later, the front edge of the bronchofiberscope 200 is set on the base 21 at the front edge of the side hole 20 as shown in lower side figure of Fig. 2.

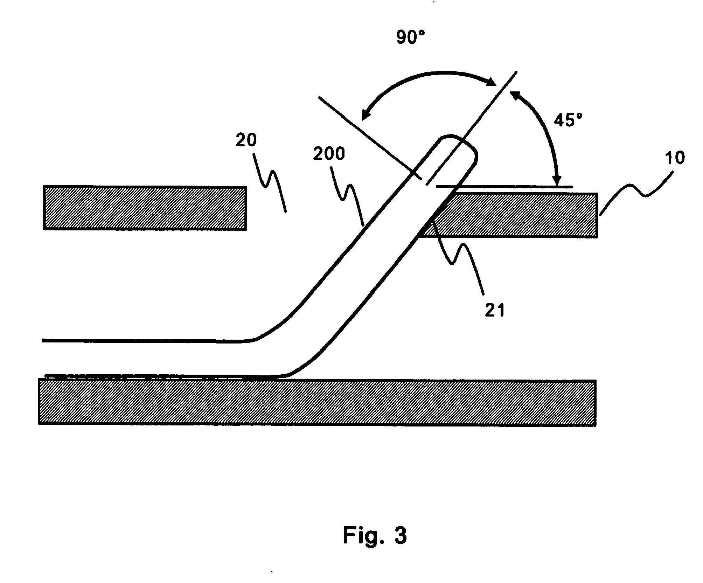

It is preferable that the oblique angle of the base 21 is from 30 degrees to 60 degrees relative to the guide tube inside wall. Fig. 3 illustrates the motion range of the front edge of the bronchofiberscope 200 set on the base 21. The base 21 shown in Fig. 3 is 45 degree relative to the guide tube inside wall. When the glottis, which is the entrance of the trachea, is observed obliquely in front from the side hole 20, the front edge of the bronchofiberscope 200 can go through the glottis easily by going forward naturally. In addition, the motion range will be expanded. For example, the bronchofiberscope 200 can bend the front edge part 90 degrees to the forward and to the backward by the wire operation. The motion range without utilizing the base 21 is from -90 degree to +90 degree. The glottis is located on the upper side when the guide tube 10 is located correctly, so the motion range for seeking the glottis is effectively limited to 0 to +90 degree. However, the front edge part of the bronchofiberscope 200 can bend 45 degree to the forward and 90 degree to the backward by utilizing the base 21, therefore the effective motion range utilizing the base 21 is from 0 degree to + 135 degree, and the motion range for the glottis observation becomes large.

Next, the procedure for the endotracheal intubation assist instrument 100 is described.

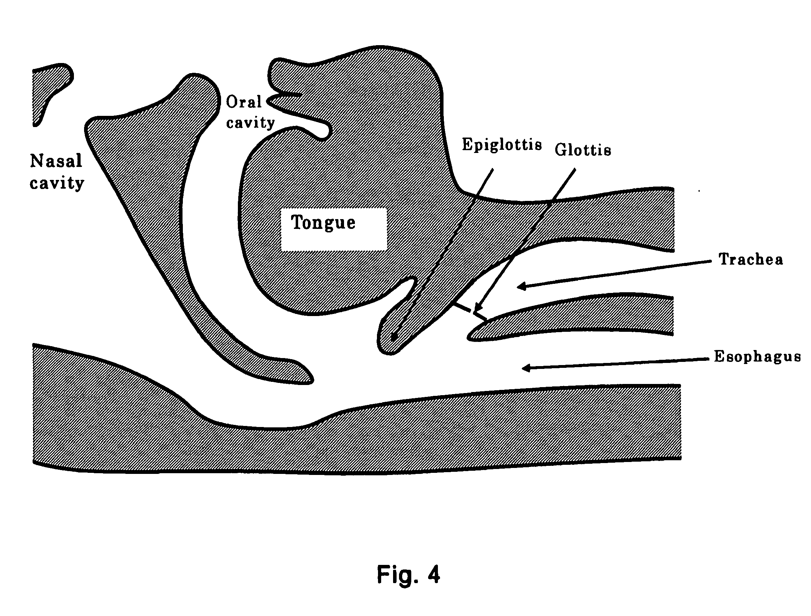

Fig. 4 illustrates the cross section view of the general structure of the oral cavity, the nasal cavity, the esophagus and the trachea. There is a tongue in the oral cavity and there is an epiglottis inside of the throat. Generally, the bronchofiberscope 200 goes forward naturally along to the organ structure and, it will reach the entrance of the esophagus either from the oral cavity or the nasal cavity trough the pharynx side or the larynx side.

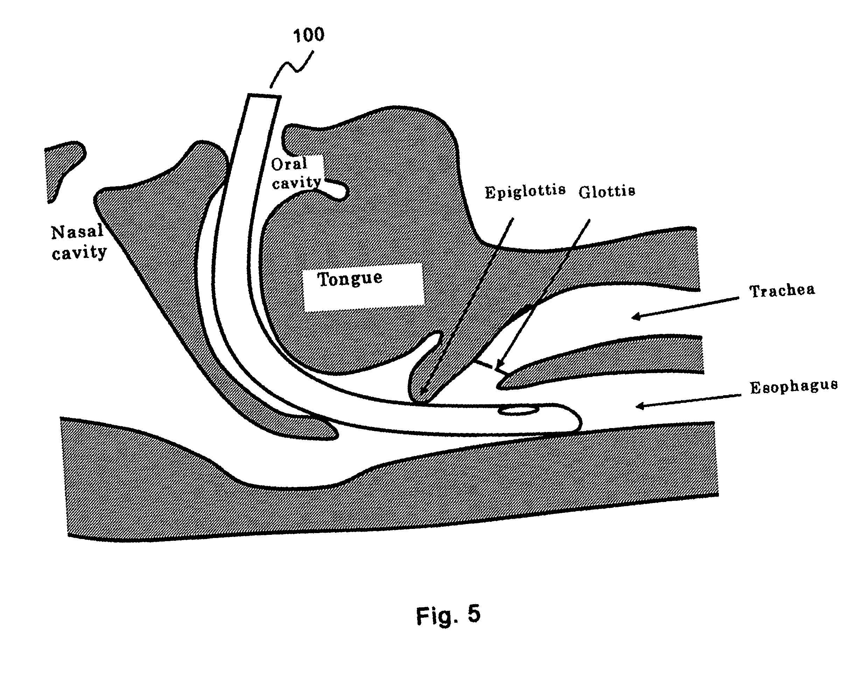

Fig. 5 illustrates the view showing the appearance when the front edge of the endotracheal intubation assist instrument 100 has reached the entrance of the esophagus via the oral cavity. The guide tube 10 goes forward naturally from the oral cavity, and the front edge of the guide tube 10 reaches the entrance of the esophagus. This state can be obtained easily just by pushing the guide tube 10 forward until the front edge reaches the entrance of the esophagus, no matter where the glottis is. The operator can operate the endotracheal intubation assist instrument 100 easily to obtain the status shown in Fig. 5. The status of the guide tube 10 inserted from the oral cavity or the nasal cavity shown in Fig. 5 does not necessarily require the positioning of the side hole 20 to face the glottis. Actually, the side hole 20 is inserted into the esophagus so the glottis is not observed from the side hole 20 shown in Fig. 5.

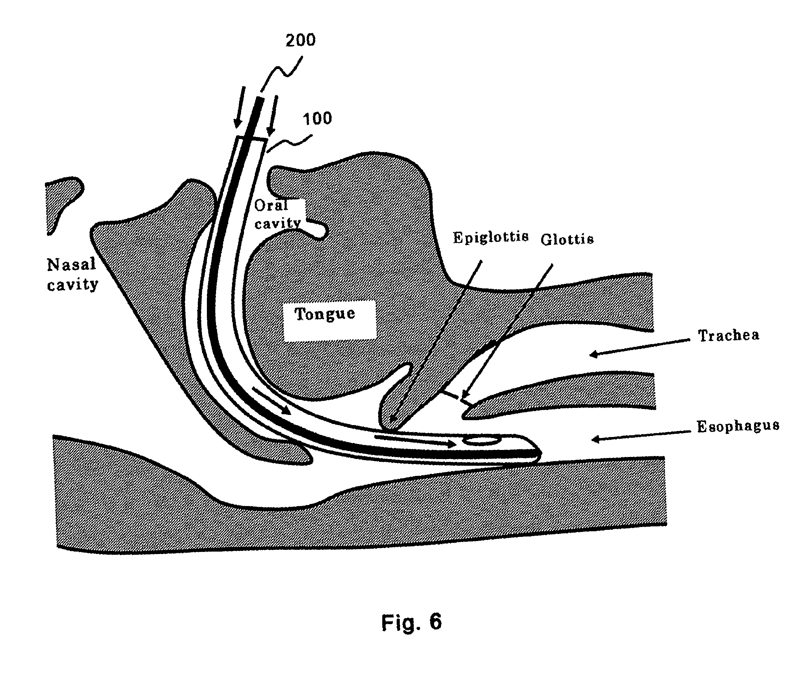

Fig. 6 illustrates the view showing the appearance when the bronchofiberscope 200 is inserted into the inside of the endotracheal intubation assist instrument 100 in the state shown in Fig. 5. The bronchofiberscope 200 is inserted from the terminal opening 11 of the guide tube 10. There is the leading opening 12 at the front edge of the guide tube 10. When the front edge of the bronchofiberscope 200 reaches the front edge of the guide tube 10, the outer view field around the front edge of the guide tube 10 is obtained through the leading opening 12. The esophagus wall can be recognized easily by observing via the bronchofiberscope 200. Therefore the operator can judge easily whether the front edge of the guide tube 10 is inserted into the entrance of the esophagus correctly or not.

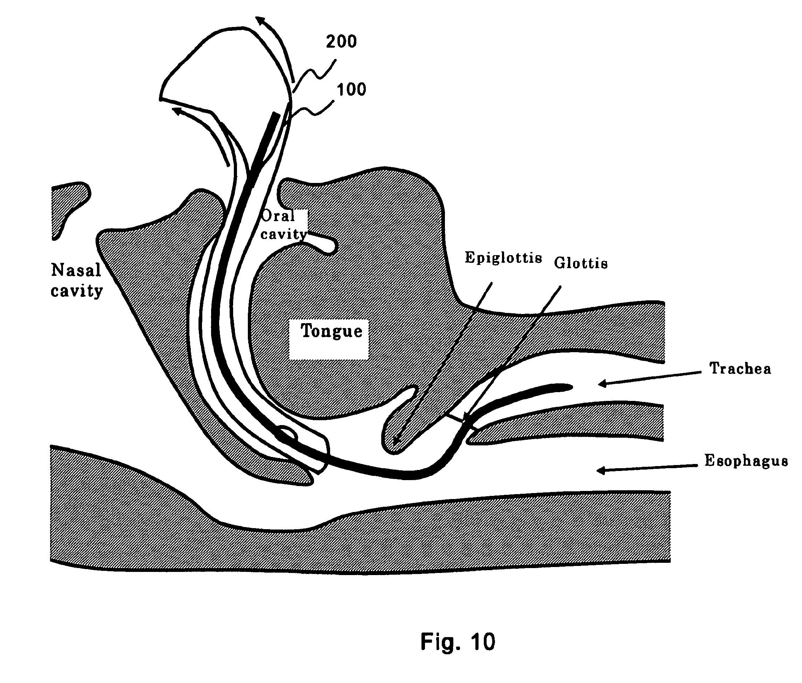

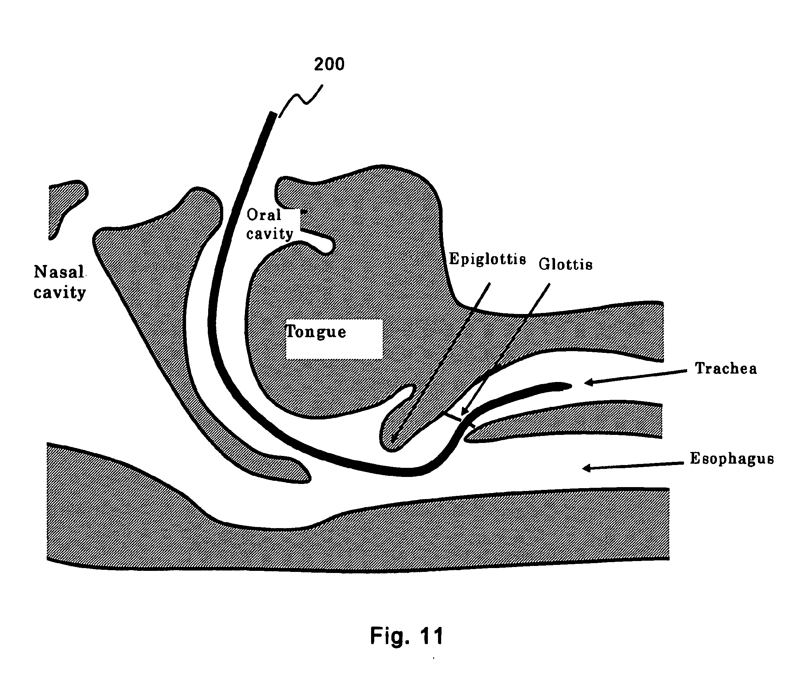

If the front edge of the guide tube 10 is inserted into the trachea via the glottis at this procedure, the operator can recognize whether the front edge of the guide tube 10 is inserted into the trachea or not. In this case, the bronchofiberscope 200 happens to be lead to the trachea at this stage, the following several procedures can be omitted, and as described later, the guide tube 10 is cut and removed as shown in Fig. 10 and Fig. 11, and the final state in which only the bronchofiberscope 200 is inserted into the trachea is achieved.

Hereinafter, the front edge of the guide tube 10 is inserted into the entrance of the esophagus as expected.

After the operator verifies the front edge of the guide tube 10 is inserted into the entrance of the esophagus by observing via the bronchofiberscope 200, he then tries to find the side hole 20 by pulling back the front edge of the bronchofiberscope 200 and put it onto the base 21.

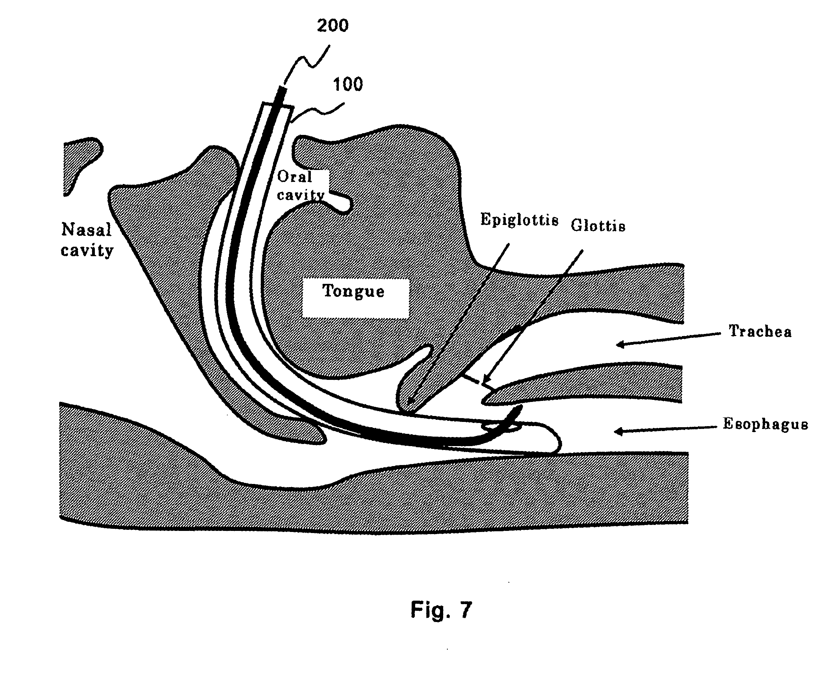

Fig. 7 illustrates the view showing the appearance when the front edge of the bronchofiberscope 200 is put onto the base 21 of the side hole 20. In this example, only the entrance of the esophagus can be observed by the view field obtained by the bronchofiberscope 200 whose front edge is put onto the base 21 of the side hole 20, and the glottis is not observed in this state.

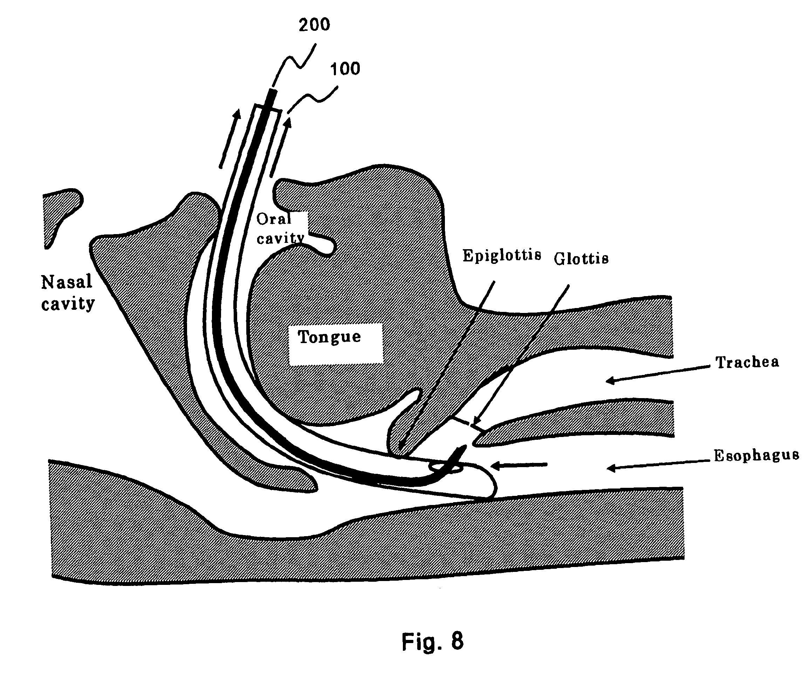

Next, the operator keeps on observing with the bronchofiberscope 200, and the guide tube 10 with the bronchofiberscope 200 shown in Fig. 7 is pulled back slowly. In this example, the view field of the bronchofiberscope 200 can cover the glottis when the guide tube 10 with the bronchofiberscope 200 is reached at the status shown in Fig. 8. When the glottis is found by the view field of the bronchofiberscope 200 whose front edge is put on the base 21, the glottis is located obliquely in front of the base 21.

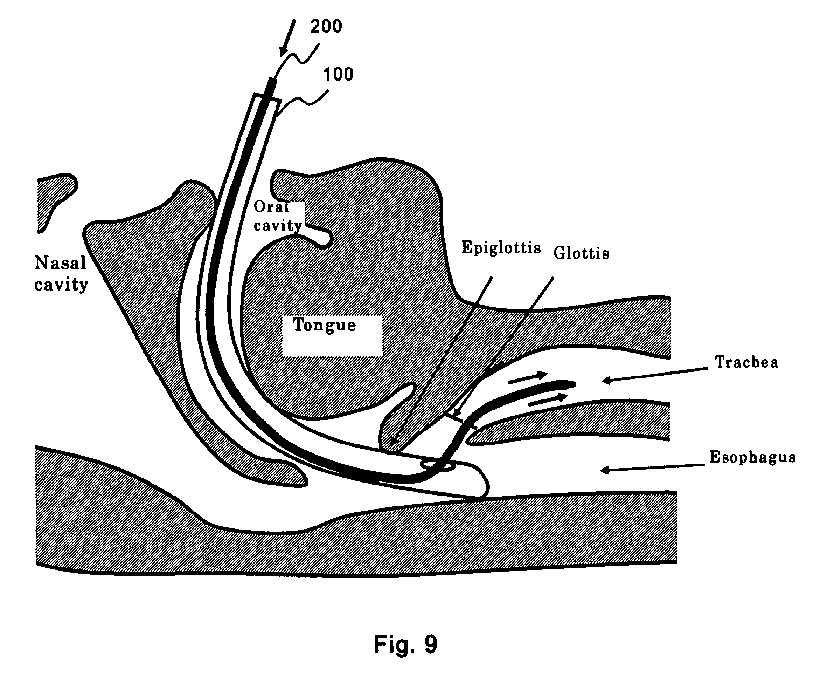

When the glottis is found by the view field of the bronchofiberscope 200, the operator operates the front edge of the bronchofiberscope 200 to go forward to the glottis as shown in Fig. 9. The base 21 is the base for supporting the front edge of the bronchofiberscope 200 and the front edge is facing to the glottis, the front edge of the bronchofiberscope 200 goes forward naturally to the glottis. The motion range of the front edge of the bronchofiberscope 200 is secured sufficiently, and it is easy to reach the glottis.

When the front edge of the bronchofiberscope 200 is inserted into the trachea, the main purpose of the endotracheal intubation assist instrument 100 of this invention is achieved.

Next, the endotracheal intubation assist instrument 100 of this invention is removed for preparing the endotracheal tube insertion to the trachea. The endotracheal intubation assist instrument 100 of this invention comprises a cut assisting structure for assisting the operator to cut the guide tube 10 from the terminal end opening up to the side hole 20 and to remove it away. In this example, the cut assisting structure is a cutting line 13 from the terminal end opening up to the side hole 20.

Fig. 10 illustrates the view showing the appearance when the guide tube 10 is cut by the cutting line 13 and only the guide tube 10 is removed and the bronchofiberscope 200 remains as it is in the trachea. If the operator keeps on handling and fixing the bronchofiberscope 200, the front edge of the bronchofiberscope 200 remains in the trachea. In this state, in order to remove only the guide tube 10, the wall of the guide tube 10 should be cut off in the following removing procedure. The cutting line 13 is installed to cut off the wall of the guide tube 10 more easily.

Fig. 11 illustrates the view showing the appearance when the guide tube 10 is removed away completely. As a result, the bronchofiberscope 200 is led from the oral cavity to the trachea.

In the final procedure, the endotracheal tube 300 is inserted into the trachea to secure the airway, and the endotracheal tube 300 is inserted by utilizing the bronchofiberscope 200 as the guide line.

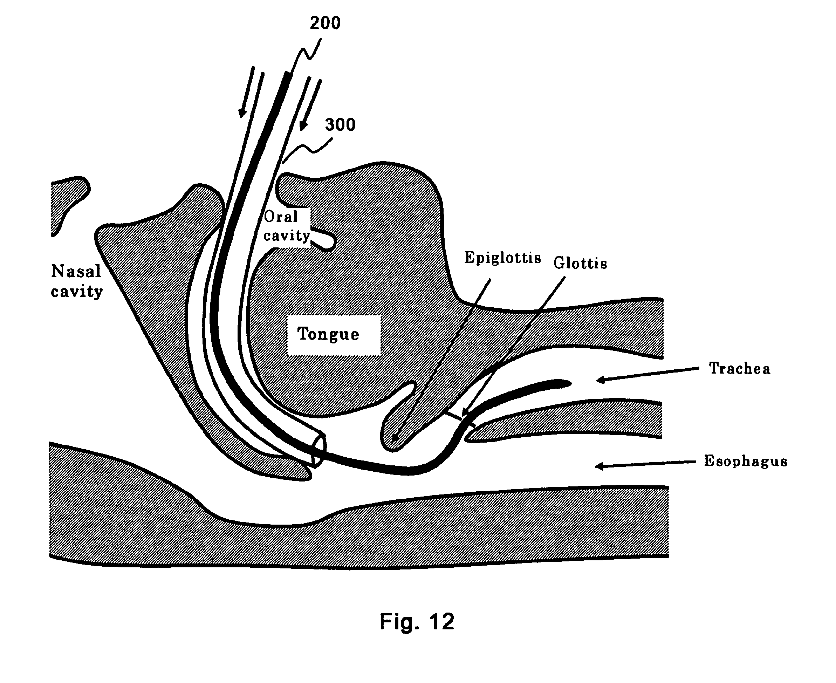

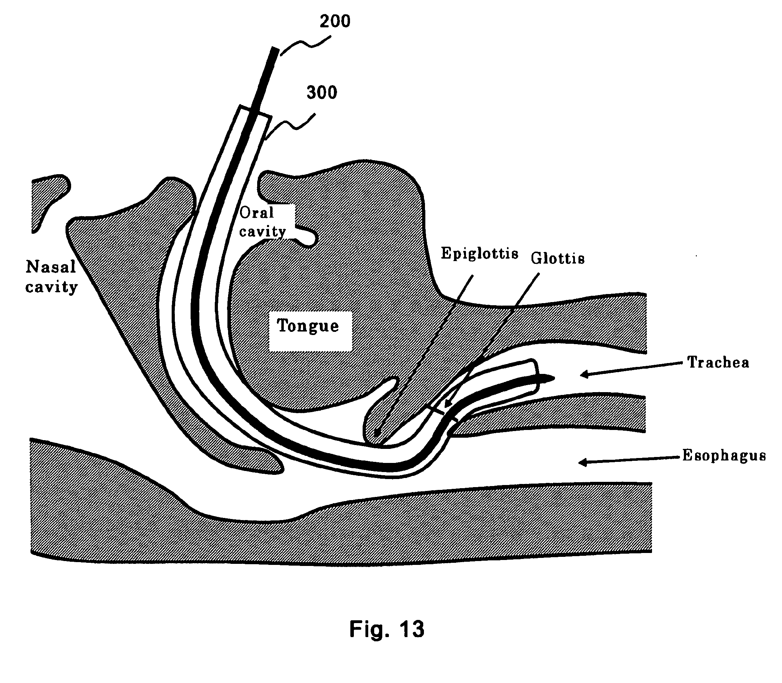

Fig. 12 illustrates the view showing the appearance when the endotracheal tube 300 is inserted by utilizing the bronchofiberscope 200 as the guide line. Fig. 13 illustrates the view showing the appearance when the endotracheal tube 300 is inserted completely

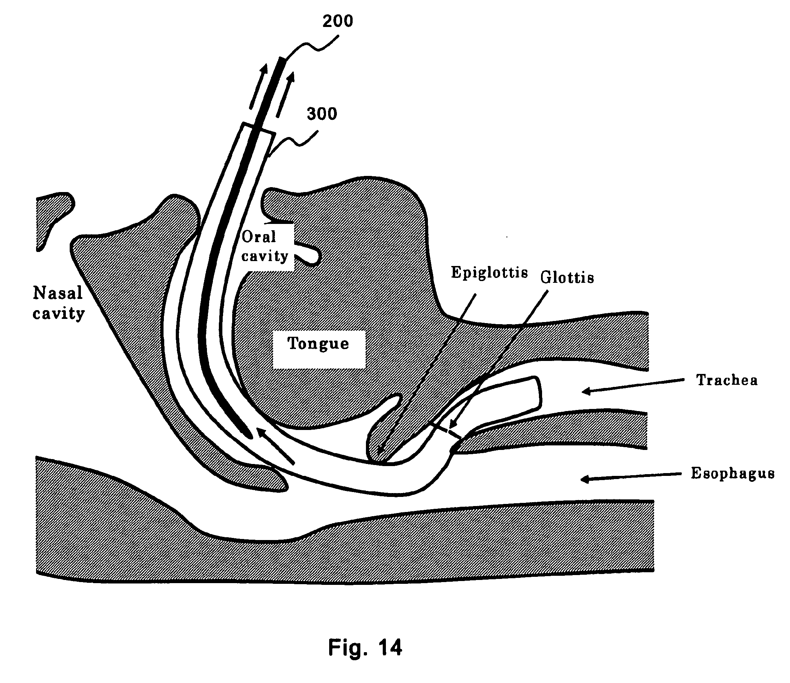

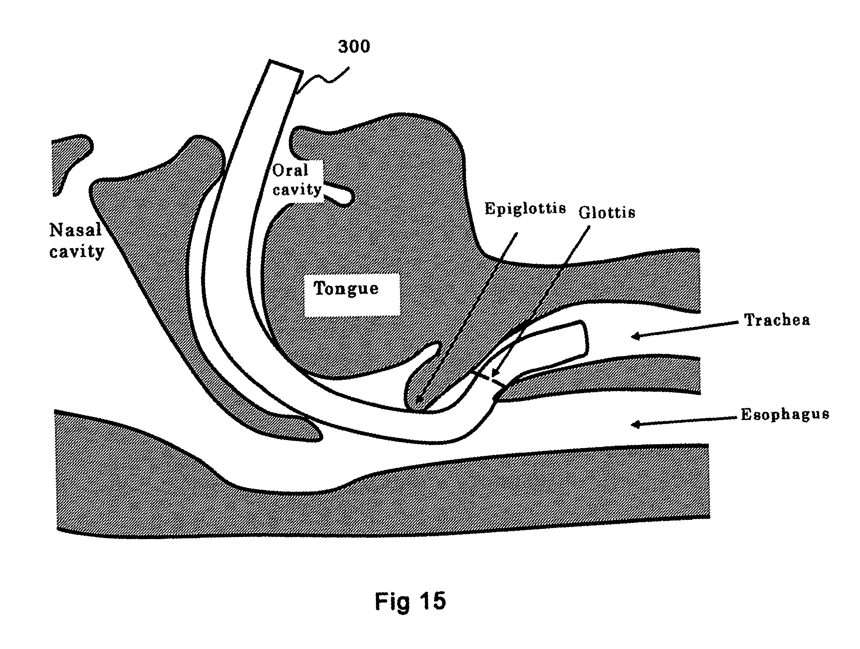

After finishing the endotracheal tube 300 insertion successfully, the bronchofiberscope 200 is removed. Fig. 14 illustrates the view showing the appearance when the bronchofiberscope 200 is removed. Fig. 15 illustrates the view showing the appearance when the bronchofiberscope 200 is removed completely and the final state of the endotracheal tube 300 insertion.

Hereinabove, the basic procedure how to use the endotracheal intubation assist instrument 100 of Embodiment 1 of the present invention is described above.

### (Embodiment 2)

The second endotracheal intubation assist instrument 100a of this Embodiment 2, representing the present invention is described below. The cut assisting structure installed in the guide tube wall of this second endotracheal intubation assist instrument 100a is different from that of the first endotracheal intubation assist instrument 100. The rest parts of this second endotracheal intubation assist instrument 100a are the same those of the first endotracheal intubation assist instrument 100. The cut assisting structure installed in the guide tube wall of this second endotracheal intubation assist instrument 100a comprises a cut gap 14 installed from the terminal end opening up to the side hole 20 and a cover film 15 to cover the cut gap 14.

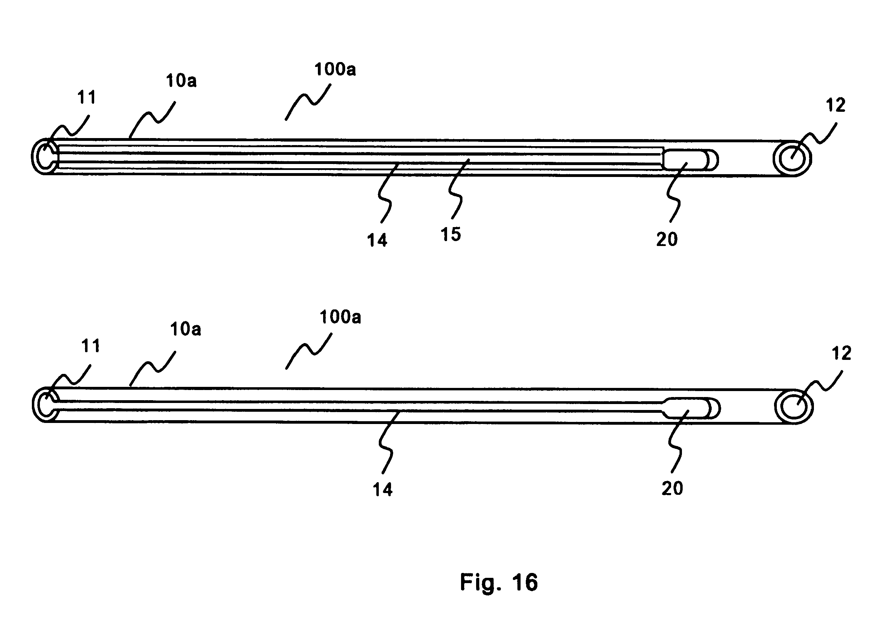

The upper figure of the Fig. 16 illustrates the cut gap 14 as the cut assisting structure and the cover film 15 covering the cut gap 14 of the second endotracheal intubation assist instrument 100a. The lower figure of the Fig. 16 illustrates the view showing the appearance that the cover film 15 is torn.

In this second endotracheal intubation assist instrument 100a of this Embodiment 2, the cut gap 14 is installed at the side wall of the guide tube 10a as an opening, however it is covered by the cover film 15. The procedures shown in the Fig. 5 to Fig. 9 of Embodiment 1 can be applied to those procedures of Embodiment 2 in the same way.

In Embodiment 1, the guide tube 10 of the first endotracheal intubation assist instrument 100 is cut by the cutting line 13 as shown in Fig. 10. However, in Embodiment 2, the guide tube 10a of the second endotracheal intubation assist instrument 100a is opened by tearing the cover film 15 and the bronchofiberscope 200 is extracted from the guide tube 10a through the cut gap 14. The guide tube 10a is separated from the bronchofiberscope 200 and only the guide tube 10a is removed away. The status after finishing removing the guide tube 10a away is the same as the status shown in Fig. 11. The procedures for inserting the endotracheal tube into the trachea are the same as those shown in Fig. 12 to Fig. 15 of Embodiment 1.

### (Embodiment 3)

The third endotracheal intubation assist instrument 100b of this Embodiment 3 helpful for understanding the present invention is described below. There is no guide line on the inner wall of the guide tube 10 of the first endotracheal intubation assist instrument 100 of Embodiment 1. However in this Embodiment 3, there is a blue-green guide line 16 indicating on the inner wall from the terminal end up to the front edge of the guide tube 10b of the third endotracheal intubation assist instrument 100b of Embodiment 3.

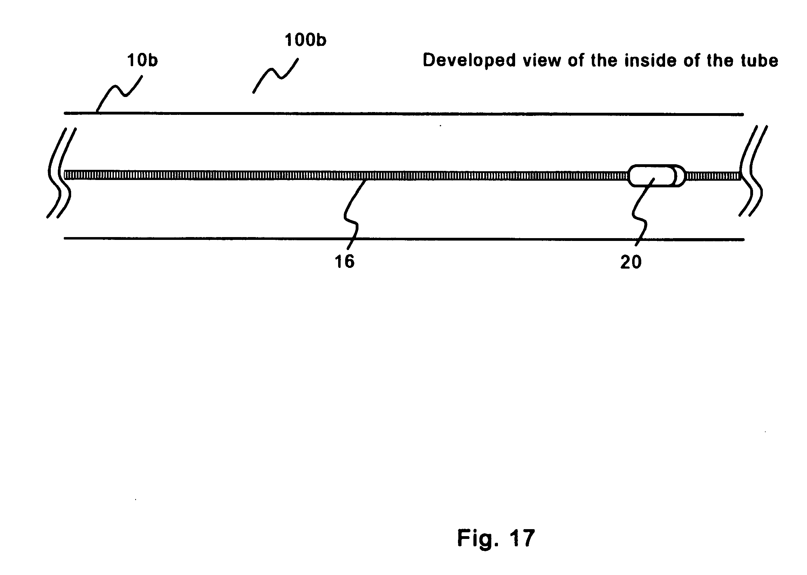

Fig. 17 illustrates the view showing the developed appearance of the inside wall of the guide tube 10b. There is a blue-green guide line 16 indicating on the inner wall from the terminal end up to the front edge of the guide tube 10b. The guide line 16 gives several merits. The first merit is that it is easy for the operator to verify the positioning status of the guide tube 10 in the patient's organs. It is assumed that the endotracheal intubation assist instrument is inserted into organs along the body midline such as the oral cavity or the nasal cavity through the pharynx, the larynx, the trachea and the esophagus. If the guide tube 10 is drawn onto the midline of the inside wall shown as Fig. 17, it is easy to recognize whether the guide tube 10 is arrayed along to midline or the guide tube 10 is curved or twisted in the patient's organs. Next, the second merit is that it is easy to find the side hole 20. It is easy for the operator to recognize the positioning status of the bronchofiberscope 200 in the guide tube 10b during the bronchofiberscope 200 inserting procedure as shown in Fig. 5 to Fig. 7. In addition the side hole 20 is always found just by following the guide line 16.

The reason why blue-green is selected as the color of the guide line 16 is that the complementary color of red is green and blue. If the blood exists in the guide tube 10b, the blue-green guide line 16 can be recognized comparably easily by the operator. For example the width of the guide line 16 is about 3 mm.

### (Embodiment 4)

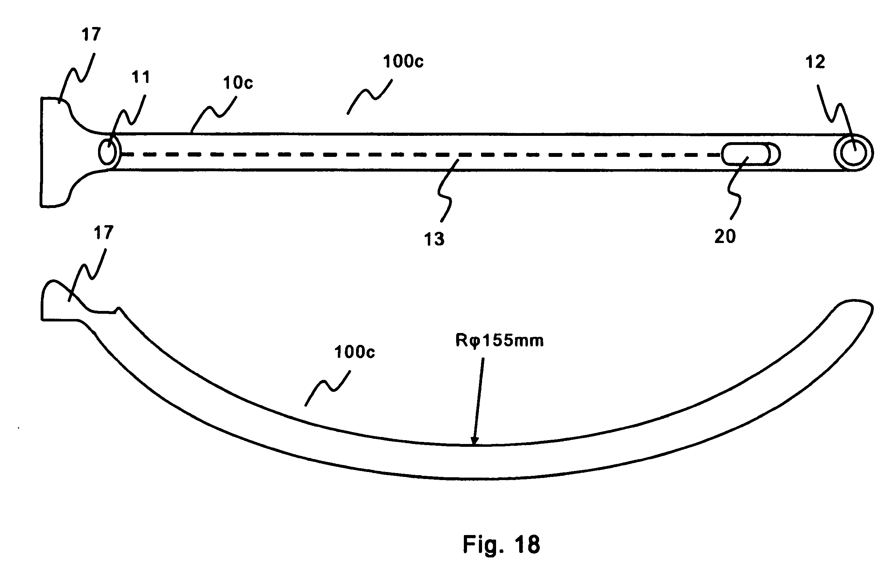

The forth endotracheal intubation assist instrument 100c of this Embodiment 4 helpful for understanding the present invention is described below. Fig. 18 illustrates a basic structure of the fourth endotracheal intubation assist instrument 100c. The first endotracheal intubation assist instrument 100 of this Embodiment 1 of the present invention, the basic figure of the guide tube is simply the tube figure even though the outline is curved and there is no grip. However, with the fourth endotracheal intubation assist instrument 100c, there is a grip 17 at the terminal end of the guide tube 10c as shown in Fig. 18. With the grip 17, the operator can grip the guide tube 10c firmly during the operation procedures of the endotracheal intubation assist instrument 100c. It become easier for the operator to perform the operation for the guide tube 10c insertion shown in Fig. 4 to Fig. 5, the operation for the side hole 20 positioning adjustment shown in Fig. 8 and the operation for the guide tube 10c removal shown in Fig. 10.

### (Embodiment 5)

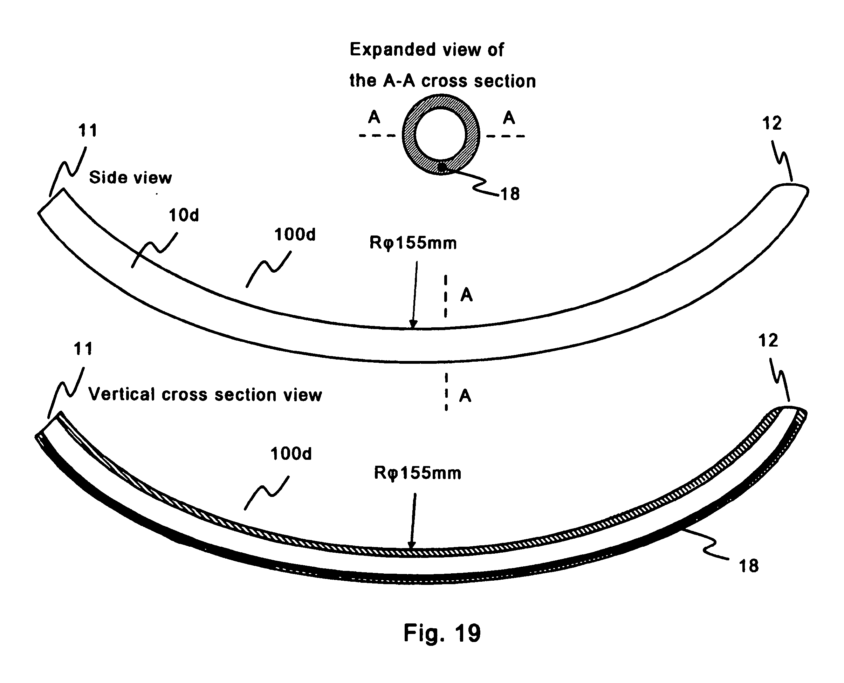

The fifth endotracheal intubation assist instrument 100d of this Embodiment 5 helpful for understanding the present invention is described below. Fig. 19 illustrates a basic structure of the fifth endotracheal intubation assist instrument 100d. Fig. 19 shows a side view of the endotracheal intubation assist instrument 100d, a vertical cross-sectional view of the endotracheal intubation assist instrument 100d, and an expanded view of A-A cross section.

For the endotracheal intubation assist instrument 100d of this Embodiment 5, the guide tube 10d is made of silicone material and the rotation torque transmitter 18 is installed into the guide tube 10d. The rotation torque transmitter 18 has appropriate axial rigidity in order to transmit the rotation torque applied to the terminal end up to the front edge. For example, the torque transmitter 18 is made of wire or plastic having appropriate axial rigidity.

It is necessary for bronchofiberscope 200 to move its view field via the side hole 11 of the guide tube 10d around the axis in order to find the glottis around the bronchia. The torque transmitter 18 transmits the rotation torque applied to the terminal end to the front edge, so the rotation operation for the front edge becomes easier. It is preferable that the guide tube 10d is made of soft and flexible material in order to protect the organs from injury during the endotracheal tube insertion procedure. However, if the axial rigidity of the guide tube 10d is insufficient, the transmitted rotation torque is not enough to operate the rotation of the front edge even though the operator applies the force to the terminal end. Therefore, as shown above, the axial rigidity of the guide tube 10d is enhanced by installing the wire or the plastic having an appropriate axial rigidity into the guide tube 10d, the operator can control the twist rotation operation more easily.

### (Embodiment 6)

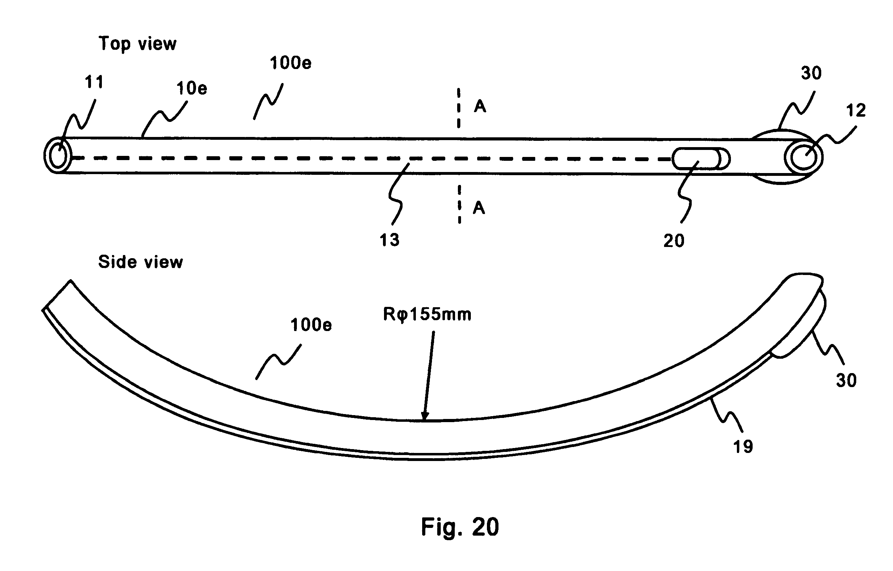

The sixth endotracheal intubation assist instrument 100e of this Embodiment 6 helpful for understanding the present invention is described below. Fig. 20 illustrates a basic structure of the sixth endotracheal intubation assist instrument 100 e. Fig. 20 shows a top view of the endotracheal intubation assist instrument 100e, a side view of the endotracheal intubation assist instrument 100e.

The endotracheal intubation assist instrument 100e of this Embodiment 6, the balloon 30 is installed around the front edge of the guide tube 10e. The guide tube 10e comprises an air tube from the terminal end up to the front edge for pumping the air into the balloon.

The balloon 30 can be the same structure as the balloon used for the catheterization treatment, the air tube 19 can be the same structure as the air tube use for the balloon pumping for the catheterization treatment, with the front edge of the air tube 19 is connected to the balloon 30.

The balloon 30 is an object which can inflate and contract near the front edge of the guide tube 10e. Therefore the angle of elevation of the front edge of the guide tube 10e against the bronchia wall can be adjusted by inflating the balloon 30.

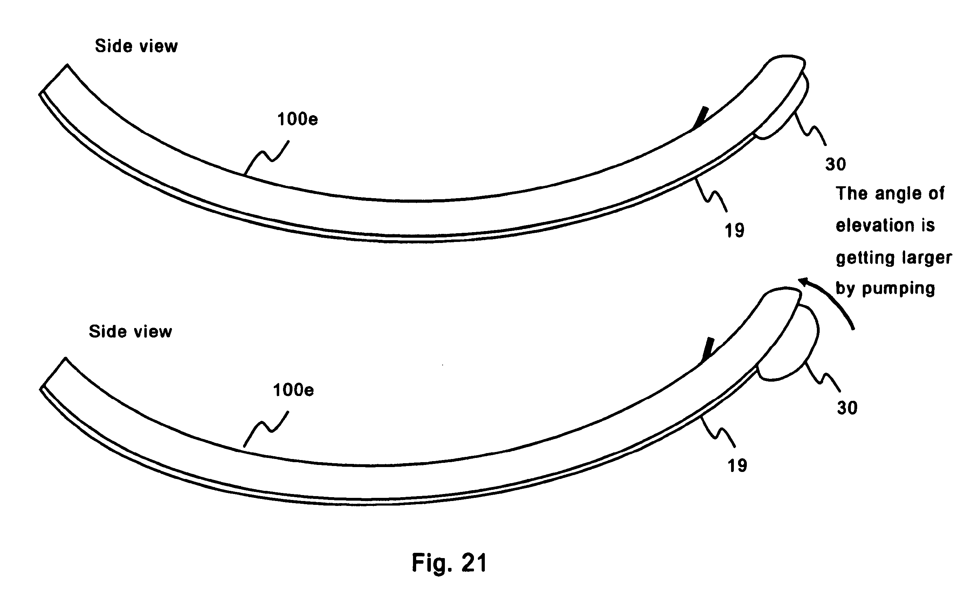

Fig. 21 illustrates the view showing the appearance when the angle of elevation of the front edge of the guide tube 10e against the bronchia wall becomes large by inflating the balloon 30 through the air tube 19. When the air is pumped into the balloon 30 via the air tube 19, the balloon 19 is inflated gradually and the volume becomes large. Thus the distance between the bronchia wall and the front edge of the guide tube 10e becomes large, and the angle of elevation of the front edge of the guide tube 10e against the bronchia wall becomes large. The view field obtained through the bronchofiberscope 200 via the side hole 20 will be moved according to the move of the angle of elevation of the front edge of the guide tube 10e against the bronchia wall.

When the trachea is difficult to observe by the bronchofiberscope 200 via the side hole 12, the view field of the bronchofiberscope 200 and the front edge of the guide tube 10e are changed together by adjusting the balloon 30 inflation in order to find the trachea easily.

### Industrial Applicability

This invention can be applied to the endotracheal intubation assist instrument for inserting the endotracheal tube for the artificial respiration into the trachea of the patient who has difficulty with the airway maintenance.

## Claims

1. An endotracheal intubation assist instrument comprising:
a guide tube (10, 10a, ..., 10e) having flexibility and a gently curved outline;
a side hole (20) arranged in a wall of the guide tube (10, 10a, ..., 10e) near the front edge on the inner circumference side of the curved outline;
wherein the endotracheal intubation assist instrument is configured such that the front edge of the guide tube (10, 10a, ..., 10e) is insertable from the oral cavity or the nasal cavity up to a vicinity of the entrance of the esophagus, a bronchofiberscope is insertable from the terminal end opening (11) of the guide tube (10, 10a, ..., 10e) up to the side hole (20) arranged near the front end of thetrachea, and the view field of the bronchofiberscope can be ensured via the side hole (20) so that the trachea can be ascertained easily,
**characterized in that** the endotracheal intubation assist instrument further comprises a cut gap (14) provided in advance in a wall of the guide tube (10, 10a, ..., 10e) from the terminal end opening (11) of the guide tube (10, 10a, ..., 10e) up to the side hole (20); so that the guide tube (10, 10a, ..., 10e) can be removed more easily after the bronchofiberscope has been inserted into the trachea via the cut gap (14), while the bronchofiberscope remains in the trachea.

2. An endotracheal intubation assist instrument according to claim 1, wherein the cut gap (14) is covered by a film (15), the guide tube (10, 10a, ..., 10e) can be removed via the cut gap (14) by cutting the film (15) and opening the cut gap (14).

3. An endotracheal intubation assist instrument according to claim 1, wherein the cut gap (14) is covered by the tube wall with a cutting line provided from the terminal end opening (11) of the guide tube (10, 10a, ..., 10e) up to the side hole (20), the cutting line being used as a cut assisting structure (13) to open the cut gap when removing the guide tube (10, 10a, ..., 10e) from the bronchofiberscope.

4. An endotracheal intubation assist instrument according to any one of claims 1 to 3, wherein the front edge of the side hole (20) has a ramp part as a base, on which the front edge of the bronchofiberscope can be set, so that the view field from the front edge of the bronchofiberscope can be ensured obliquely in front of the side hole (20).

5. An endotracheal intubation assist instrument according to claim 4, wherein the oblique angle of the base is from 30 degree to 60 degree relative to the guide tube axis direction.

6. An endotracheal intubation assist instrument according to any one of claims 1 to 5, comprising a blue-green guide line (16) extending from the terminal end up to the front edge on the inner wall of the guide tube (10, 10a, ..., 10e).

7. An endotracheal intubation assist instrument according to any one of claims 1 to 6, further comprising a grip (17) at the terminal end of the guide tube (10, 10a, ..., 10e).

8. An endotracheal intubation assist instrument according to any one of claims 1 to 7, wherein the position of the side hole (20) on the guide tube (10, 10a, ..., 10e) is about 30 mm to 80 mm from the front edge.

9. An endotracheal intubation assist instrument according to any one of claims 1 to 8, wherein the length of the side hole (20) of the guide tube (10, 10a, ..., 10e) is about 10 mm to 30 mm along the axis direction.

10. An endotracheal intubation assist instrument according to any one of claims 1 to 10, wherein the guide tube (10, 10a, ..., 10e) further comprises a rotation torque transmitter (18) that can transmit rotation torque of the force applied to the terminal end of the guide tube (10, 10a, ..., 10e) to the front edge.

11. An endotracheal intubation assist instrument according to any one of claims 1 to 10, further comprising an inflatable/deflatable balloon (30) arranged near the front edge of the guide tube (10, 10a, ..., 10e), wherein the angle of elevation of the front edge of the guide tube (10, 10a, ..., 10e) against the bronchia wall can be adjusted by inflating the balloon (30).

## Patentansprüche

1. Hilfsinstrument für endotracheale Intubation, aufweisend:
einen flexiblen Führungsschlauch (10, 10a, ..., 10e) mit schwach gekrümmtem Umriss;
ein seitliches Loch (20), welches in einer Wand des Führungsschlauchs (10, 10a, ..., 10e) in der Nähe des vorderen Randes auf der inneren umlaufenden Seite des gekrümmten Umrisses angeordnet ist;
wobei das Hilfsinstrument für endotracheale Intubation so eingerichtet ist, dass der vordere Rand des Führungsschlauchs (10, 10a, ..., 10e) von der Mundhöhle oder der Nasenhöhle aus bis in die Nähe des Eingangs der Speiseröhre einführbar ist,
wobei ein Bronchofiberskop von einer terminalen Endöffnung (11) des Führungsschlauchs (10, 10a, ..., 10e) bis zu dem seitlichen Loch (20), welches nahe dem vorderen Ende der Luftröhre angeordnet ist, einführbar ist, und das Blickfeld des Bronchofiberskops durch das seitliche Loch (20) sichergestellt werden kann, so dass die Luftröhre einfach bestimmt werden kann,
**dadurch gekennzeichnet, dass** das Hilfsinstrument für endotracheale Intubation weiterhin einen Schnittspalt (14) aufweist, welcher im Vorhinein in einer Wandung des Führungsschlauchs (10, 10a, ..., 10e) von der terminalen Endöffnung (11) des Führungsschlauchs (10, 10a, ..., 10e) bis zum seitlichen Loch (20) vorgesehen ist, so dass der Führungsschlauch (10, 10a, ..., 10e) mit Hilfe des Schnittspalts (14) einfacher entfernt werden kann nachdem das Bronchofiberskop in die Luftröhre eingeführt wurde, wobei das Bronchofiberskop in der Luftröhre verbleibt.

2. Hilfsinstrument für endotracheale Intubation nach Anspruch 1, wobei der Schnittspalt (14) mit einem Film (15) bedeckt ist, und der Führungsschlauch (10, 10a, ..., 10e) mit Hilfe des Schnittspalts (14) entfernt werden kann, indem der Film (15) zerschnitten und der Schnittspalt (14) geöffnet wird.

3. Hilfsinstrument für endotracheale Intubation nach Anspruch 1, wobei der Schnittspalt (14) durch eine Schlauchwand mit einer Schnittlinie verdeckt ist, die von der terminalen Endöffnung (11) des Führungsschlauchs (10, 10a, ..., 10e) bis zum seitlichen Loch (20) vorgesehen ist, wobei die Schnittlinie als Schnitthilfsstruktur (13) verwendet werden kann, um den Schnittspalt zu öffnen, wenn der Führungsschlauch (10, 10a, ..., 10e) vom Bronchofiberskop entfernt wird.

4. Hilfsinstrument für endotracheale Intubation nach einem der Ansprüche 1 bis 3, wobei der vordere Rand des seitlichen Lochs (20) einen Rampenabschnitt als Basis aufweist, auf welchem der vordere Rand des Bronchofiberskop aufsetzbar ist, so dass das Blickfeld vom vorderen Rand des Bronchofiberskops schräg vor dem seitlichen Loch (20) gewährleistet werden kann.

5. Hilfsinstrument für endotracheale Intubation nach Anspruch 4, wobei der Abschrägwinkel von der Basis 30 bis 60 Grad relativ zur Richtung der Schlauchachse beträgt.

6. Hilfsinstrument für endotracheale Intubation nach einem der Ansprüche 1 bis 5, weiterhin aufweisend eine blaue bzw. grüne Führungslinie (16), die vom terminalen Ende bis zum vorderen Rand an der inneren Wand des Führungsschlauchs (10, 10a, ..., 10e) verläuft.

7. Hilfsinstrument für endotracheale Intubation nach einem der Ansprüche 1 bis 6, weiterhin aufweisend einen Griff (17) am terminalen Ende des Führungsschlauchs (10, 10a, ..., 10e).

8. Hilfsinstrument für endotracheale Intubation nach einem der Ansprüche 1 bis 7, wobei die Position des seitlichen Lochs (20) im Führungsschlauch (10, 10a, ..., 10e) etwa 30 mm bis 80 mm vom vorderen Rand ist.

9. Hilfsinstrument für endotracheale Intubation nach einem der Ansprüche 1 bis 8, wobei die Länge des seitlichen Lochs (20) im Führungsschlauch (10, 10a, ..., 10e) etwa 10 mm bis 30 mm entlang der axialen Richtung beträgt.

10. Hilfsinstrument für endotracheale Intubation nach einem der Ansprüche 1 bis 10, wobei der Führungsschlauch (10, 10a, ..., 10e) weiterhin einen Drehomementübertrager (18) aufweist, welcher ein Drehmoment der auf das terminale Ende des Führungsschlauchs (10, 10a, ..., 10e) ausgeübten Kraft an den vordere Rand übertragen kann.

11. Hilfsinstrument für endotracheale Intubation nach einem der Ansprüche 1 bis 10, ferner aufweisend einen aufblasbaren/entleerbaren Ballon (30), der nahe dem vorderen Rand des Führungsschlauchs (10, 10a, ..., 10e) angeordnet ist, wobei der Erhebungswinkel des vorderen Rands des Führungsschlauchs (10, 10a, ..., 10e) gegen die Bronchienwand bzw. Luftröhrenwand durch Aufblasen des Ballons (30) eingestellt werden kann.

## Revendications

1. Instrument aidant à insérer un tube trachéal comprenant:
un tube de guidage (10, 10a, ..., 10e) étant flexible et un contour légèrement incurvé;
un orifice latéral (20) disposé dans une paroi du tube de guidage (10, 10a, ..., 10e) à proximité du bord avant sur le côté de la circonférence interne du contour incurvé;
dans lequel l'instrument aidant à insérer un tube trachéal est conçu de telle sorte que le bord avant du tube de guidage (10, 10a, ..., 10e) peut être inséré à partir de la cavité orale ou la cavité nasale jusqu'à une proximité de l'entrée de l'oesophage, un bronchofibroscope peut être inséré à partir de l'orifice d'extrémité terminal (11) du tube de guidage (10, 10a, ..., 10e) jusqu'à l'orifice latéral (20) disposé à proximité de l'extrémité avant de la trachée, et le champ de visualisation du bronchofibroscope peut être garanti à travers l'orifice latéral (20) de telle sorte que la trachée peut être facilement vérifiée,
**caractérisé par le fait que** l'instrument aidant à insérer un tube trachéal comprend un espace de découpe (14) fourni à l'avance dans une paroi du tube de guidage (10, 10a, ..., 10e) allant de l'orifice d'extrémité terminal (11) du tube de guidage (10, 10a, ..., 10e) jusqu'à l'orifice latéral (20) ; de telle sorte que le tube de guidage (10, 10a, ..., 10e) peut être retiré plus facilement après l'insertion du bronchofibroscope dans la trachée par l'espace de coupe (14), tandis que le bronchofibroscope reste dans la trachée.

2. Instrument aidant à insérer un tube trachéal selon la revendication 1, dans lequel l'espace de découpe (14) est couvert d'un film (15), le tube de guidage (10, 10a, ..., 10e) peut être retiré par l'espace de découpe (14) en coupant le film (15) et en ouvrant l'espace de découpe (14).

3. Instrument aidant à insérer un tube trachéal selon la revendication 1, dans lequel l'espace de découpe (14) est couvert par la paroi de tube avec une ligne de découpe fournie à partir de l'orifice d'extrémité terminal (11) du tube de guidage (10, 10a, ..., 10e) jusqu'à l'orifice latéral (20), la ligne de découpe étant utilisée comme une structure d'aide à la découpe (13) pour ouvrir l'espace de découpe pendant le retrait du tube de guidage (10, 10a, ..., 10e) du bronchofibroscope.

4. Instrument aidant à insérer un tube trachéal selon l'une quelconque des revendications 1 à 3, dans lequel une partie de rampe constitue une base du bord avant de l'orifice latéral (20), sur laquelle le bord avant du bronchofibroscope peut être installé, de telle sorte que le champ de visualisation depuis le bord avant du bronchofibroscope peut être garanti en oblique devant l'orifice latéral (20).

5. Instrument aidant à insérer un tube trachéal selon la revendication 4, dans lequel l'angle oblique de la base se situe entre 30 degrés et 60 degrés par rapport à la direction de l'axe du tube de guidage.

6. Instrument aidant à insérer un tube trachéal selon l'une quelconque des revendications 1 à 5, comprenant une ligne de guidage bleu-vert (16) s'étendant de l'extrémité terminale au bord avant sur la paroi interne du tube de guidage (10, 10a, ..., 10e).

7. Instrument aidant à insérer un tube trachéal selon l'une quelconque des revendications 1 à 6, comprenant en outre une prise (17) au niveau de l'extrémité terminale du tube de guidage (10, 10a, ..., 10e).

8. Instrument aidant à insérer un tube trachéal selon l'une quelconque des revendications 1 à 7, dans lequel la position de l'orifice latéral (20) sur le tube de guidage (10, 10a, ..., 10e) se situe à environ 30 mm à 80 mm par rapport au bord avant.

9. Instrument aidant à insérer un tube trachéal selon l'une quelconque des revendications 1 à 8, dans lequel la longueur de l'orifice latéral (20) du tube de guidage (10, 10a, ..., 10e) se situe à environ 10 mm à 30 mm le long de la direction de l'axe.

10. Instrument aidant à insérer un tube trachéal selon l'une quelconque des revendications 1 à 10, dans lequel le tube de guidage (10, 10a, ..., 10e) comprend en outre un émetteur de couple de rotation (18) qui peut transmettre un couple de rotation de la force appliquée à l'extrémité terminale du tube de guidage (10, 10a, ..., 10e) au bord avant.

11. Instrument aidant à insérer un tube trachéal selon l'une quelconque des revendications 1 à 10, comprenant en outre un ballon (30) gonflable/dégonflable disposé à proximité du bord avant du tube de guidage (10, 10a, ..., 10e), dans lequel l'angle d'élévation du bord avant du tube de guidage (10, 10a, ..., 10e) contre les parois bronchiales peut être ajusté en gonflant le ballon (30).
